# EUROPEAN PATENT SPECIFICATION

(11) **EP 4 202 437 B1**
(45) Date of publication and mention of the grant of the patent: **02.12.2026**
(21) Application number: 21857977.9
(22) Date of filing: 05.04.2021
(51) Int. Cl.: G06T 7/00, G01N 33/36

(54) **SYSTEM FOR EVALUATING DISTRIBUTION OF FIBER BUNDLES IN FIBER-REINFORCED MATERIAL**
SYSTEM ZUR BEWERTUNG DER VERTEILUNG VON FASERBÜNDELN IN EINEM FASERVERSTÄRKTEN MATERIAL
SYSTÈME D'ÉVALUATION DE LA DISTRIBUTION DE FAISCEAUX DE FIBRES DANS UN MATÉRIAU RENFORCÉ PAR DES FIBRES

(30) Priority: 18.08.2020 JP 2020137884
(43) Date of publication of application: 28.06.2023
(73) Proprietor: IHI Corporation, Tokyo 135-8710 (JP)
(72) Inventor: KIMURA, Naohiro, Tokyo 135-8710 (JP)
(74) Representative: MERH-IP Matias Erny Reichl Hoffmann Patentanwälte PartG mbB
(86) International application number: PCT/JP2021/014487
(87) International publication number: WO 2022/038825

(56) References cited:
- WO-A1-2014/080622
- WO-A1-2016/052489
- JP-A- 2008 122 178
- JP-A- 2017 156 271
- JP-A- 2017 507 327
- JP-A- H11 254 545
- US-A1- 2016 349 193
- WEISSENBÖCK JOHANNES ET AL: "Comparative Visualization of Orientation Tensors in Fiber-Reinforced Polymers", vol. 23(2), 9 February 2018 (2018-02-09), XP093192483, Retrieved from the Internet <URL:https://www.ndt.net/?id=21912>

## Description

### TECHNICAL FIELD

The disclosure herein relates to a system for evaluating a distribution of fiber bundles in a fiber reinforced material.

### BACKGROUND ART

Various types of so-called fiber reinforced materials have been proposed, in which high-strength fibers (reinforcement fibers) are combined with matrices. Ceramic matrix fiber reinforced composites (CMCs), in which both the reinforcement fibers and the matrices are formed of ceramics, attract attention particularly for aeronautic application in light of its light-weight, heat resistance and strength.

A CMC is, in general, produced by first weaving reinforcement fibers into a fabric, and thereafter applying chemical vapor infiltration (CVI), liquid phase infiltration (particularly polymer impregnation and pyrolysis (PIP), for example), solid phase infiltration (SPI), or melt impregnation (MI) to the fabric to form a matrix of a ceramic combining the fabric. To give a three-dimensional structure to the CMC, the fabric should be in advance molded in a shape approximating to a desired final shape in parallel with the matrix formation.

Properties of the reinforced material are neither necessarily isotropic nor uniform. A distribution of the reinforcement fibers, particularly directions where the fibers run, strongly affects the properties, particularly strength, of the reinforced material. Generally, higher strength can be expected as it's closer to a direction where the reinforcement fibers run and further as the fibers are denser. It is thus desired to establish any method to evaluate a distribution of fiber bundles within a fiber reinforced material for the purpose of material assessment and pursuit of better processes.

The patent literatures 1 and 2 disclose related arts. Additionally, PTL 3 discloses a method and apparatus for non-destructively examining layered fiber composite components, particularly those with woven or laid fabric plies, wherein the teaching uses CT scanning to collect volumetric data and analyzes local material properties layer-by-layer in defined analysis regions. Overall, it automatically identifies ply boundaries by detecting discontinuities in material properties along the thickness direction, then averages properties within each detected ply. Non-Patent literature 1 presents an interactive visualization tool for comparing fiber orientations in short-fiber-reinforced polymers (SFRPs) between simulation data and real X-ray computed tomography (XCT) scans, wherein the tool uses superquadric tensor glyphs and heat maps to visualize and compare a degree of orientation, a cosine fiber orientation similarity, and a tensor correlations. Overall, this helps to validate simulation models against actual manufactured parts by identifying regions where fiber orientations match or differ.

### Citation List

### Patent Literature

PTL 1: WO 2014/080622 A1
PTL 2: WO 2016/052489 A1
PTL 3: US 2016 / 349 193 A1

### Non-Patent Literature

nPTL1: Weissenböck, J. et al. (2018): Comparative Visualization of Orientation Tensors in Fiber-Reinforced Polymers. In: 8th Conference on Industrial Computed Tomography (iCT 2018), Wels, Austria.

### SUMMARY

### PROBLEMS TO BE SOLVED

Where the fabric of the reinforcement fibers is kept flat in the fiber reinforced material, its distribution could be readily presumed because no part of the fiber bundles falls into disorder. Some problems would occur, however, when the fabric were to be molded so as to form a three-dimensional structure. Specifically, reinforcement fibers of ceramics, as being poorly stretchy, cannot stretch and contract enough to absorb deformation caused by bending the fabric and thus the fiber bundles would be unexpectedly displaced to create non-negligible disorder. As such disorder may cause the reinforcement fibers to run in unintended directions, insights about directions of the reinforcement fibers within the fiber reinforced material are extremely important.

### MEANS FOR SOLVING PROBLEMS

The invention is set out in the appended set of claims. Preferable embodiments are defined in the dependent claims.

### ADVANTAGEOUS EFFECTS

By calculating the reference vector data obtained by averaging data in each cell, it is enabled to carry out quantitative comparison among fiber reinforced materials in which fiber bundles are distinctly placed.

### BRIEF DESCRIPTION OF DRAWINGS

FIG. 1 is a perspective view schematically depicting a three-dimensionally formed reinforcement fiber fabric.
FIG. 2 is a perspective view schematically depicting a reinforcement fiber bundle.
FIG. 3 is a perspective view of a schematic member of a fiber reinforced material.
FIG. 4 is a perspective view schematically depicting a fabric at a simply bent part.
FIG. 5 is a perspective view schematically depicting a fabric at a complicatedly bent part and three-dimensional vectors along its respective fiber bundles.
FIG. 6 is a perspective view schematically depicting three-dimensional cells and three-dimensional vectors belonging to one of the cells.

### DESCRIPTION OF EMBODIMENTS

Exemplary embodiments will be described hereinafter with reference to the appended drawings.

In some of the drawings, reference signs "X", "Y" and "Z" mean respective axial directions in a three-dimensional coordination system. Throughout the following descriptions and appended claims, references to these directions are merely for explanatory convenience and not limiting to the embodiments. Further, the respective axial directions are not necessarily perpendicular to each other.

The disclosure hereinafter relates to a method for evaluating a distribution of fiber bundles in a fiber reinforced material by three-dimensional vector data of the fiber bundles. This method can be partially manually but substantially automatically executed when using a system with a computer and a display. A generally available and also widely used computer is applicable to the system and is provided with a storage such as a hard disc drive or a solid state drive, a temporary memory such as a random access memory, and a computing device to, by combining algorithms according to the following disclosure, execute the following processes and then express results on a display. The storage can store not only programs for executing the algorithms but also other data used in computing or for comparison.

A fiber reinforced material is in general a complex in which high-strength fibers (reinforcement fibers) are combined with a matrix. The reinforcement fibers are of a ceramic such as graphite, boron nitride or silicon carbide for example. Or, they may be of a resin such as Aramid or of any proper metal or alloy. The matrix is of a thermosetting resin, a thermoplastic resin, or a proper ceramic such as silicon carbide. That formed of a matrix of a ceramic is particularly referred to as "ceramic matrix composite" (CMC).

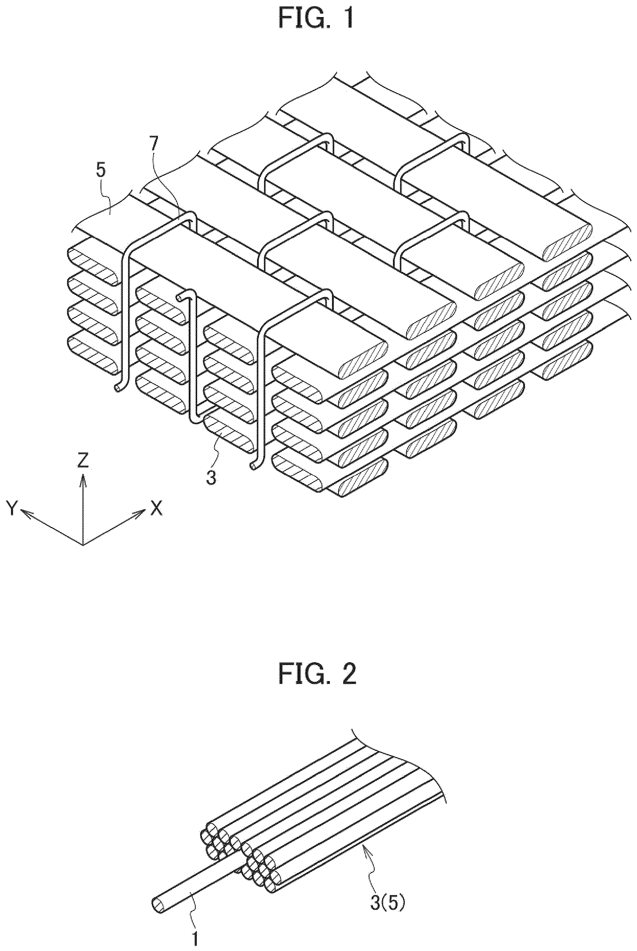

Referring mainly to FIG. 1, to the fiber reinforced material according to the present embodiment applicable is a three-dimensional reinforcement fiber fabric in which reinforcement fibers are woven into a three-dimensional structure for example. In the three-dimensional reinforcement fiber fabric, the fibers run not only in the X- and Y-directions but also in the Z-direction and loosely bind each other. According to one example, fiber bundles 3, 5 run in the X-, Y-directions respectively and are mutually layered, and fiber bundles 7 running in the Z-direction combine them together, thereby forming a three-dimensional reinforcement fiber fabric. This is of course no more than an example. These fibers may be not rendered perpendicular to each other but may be obliquely woven, or the fabric may be a two-dimensional fabric, in which the fibers are not layered in the thickness direction.

Referring to FIG. 2, the fiber bundles 3, 5, 7 are respectively those in which plural reinforcement fibers 1 running substantially in parallel are bundled together. Or, each fiber bundle 7 among them may be a single fiber. In the drawn example, the reinforcement fibers 1 are illustrated to be straight but may be twisted together. One fiber bundle 3, 5, 7 includes from 500 to 800 reinforcement fibers 1 bundled together, while the number may be arbitrarily increased or decreased.

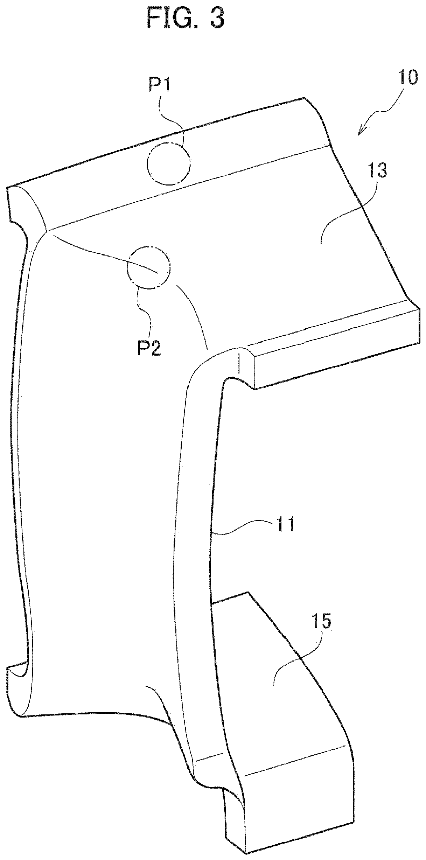

The reinforcement fiber fabric is molded into a proper shape by any known molding method such as press-forming or bagging. This is followed by, or in parallel, infiltrating a matrix precursor therein and curing them to form the matrix, thereby producing the fiber reinforced material. A product 10 illustrated in FIG. 3, which is formed of the fiber reinforced material, is a stator vane to constitute a turbine nozzle of a gas turbine engine. This is of course for explanatory convenience, and therefore various products can be produced from the fiber reinforced material.

In the turbine stator vane, a vane section 11 forms a so-called airfoil shape to express a curvature, and an outer band section 13 and an inner band section 15 are, approximately at a right angle, bent from the vane section 11. The outer band section 13 and the inner band section 15 respectively have further bent sections. Needless to say, the fabric embedded in the turbine stator vane is deformed to follow such an outline and, as discussed above, the reinforcement fiber bundles are displaced so as to enable such deformation. Therefore, even though the reinforcement fiber bundles in the original fabric were arranged orderly, the distribution and the directions of the reinforcement fiber bundles after molding would be nonuniform and uneven.

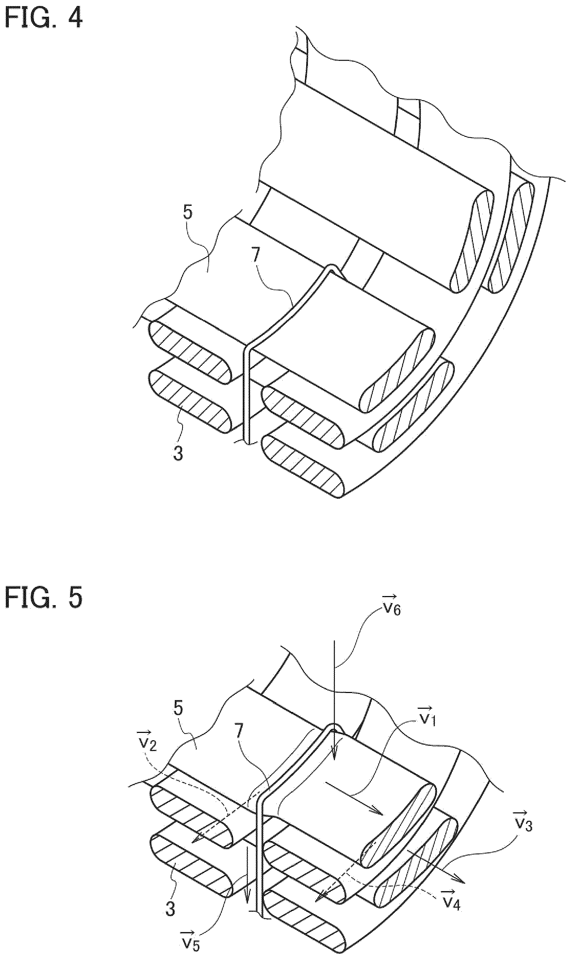

At a portion to which a sign P1 is attached in FIG. 3 for example, as the fabric is merely given a simple curve that is approximately perpendicular to the fiber bundles, as illustrated in FIG. 4, the fiber bundles could respectively warp along the curve and therein displace only slightly relative to each other. The displacement of the fiber bundles in this portion is not noticeable.

At any complicatedly bent portion, like a portion to which a sign P2 is attached in FIG. 3, where the fabric is required to curve in various directions and the curvature is necessarily steep, the fiber bundles are required not only to warp individually but also to be displaced relative to each other because otherwise they cannot absorb induced deformation. In the example shown in FIG. 5, the fiber bundles 3 must change mutual spaces and thus lose parallelism. Further, the relative height between neighboring fiber bundles 3 becomes uneven and, as reflecting it, the fiber bundles 5 crossing them undulate in the Z-axis direction.

In assessing properties of a fiber reinforced material, it is important to gain insights about the distribution of the fiber bundles inside, particularly directions where they run, and greater importance would be attached in regard to portions like the complicatedly bent portion with the sign P2.

The distribution and the directions of the fiber bundles in the fiber reinforced material can be three-dimensionally observed by using any proper interior observation means. X-ray computerized tomography (X-ray CT) is applicable even to an optically opaque substance such as CMC for example. Or, in a case of a fiber reinforced material with a transparent resin, optical means are possibly applied. Of course in place of or in addition to these means, any interior observation means using any other particles could be used.

Obtained three-dimensional observation results can be generally subject to any qualitative analysis but are barely adapted to quantitative analysis. It is, however, possible to extract vector data about the fiber bundles by applying proper publicly-known image analysis means. These obtained vector data are used in this embodiment. While FIG. 5 selectively shows some of included fiber bundles, vectors respectively parallel to these bundles are schematically attached to the drawing. The extracted data are a set of such vectors.

The extracted vector data are a set of a very large number of data and are, unless processed, inconvenient for comparison with any standard that is calculated or supposed in any way for example. Thus in the present embodiment, the extracted vector data set is processed in a way as described below and converted into a data set comparable with another data set. Such processing is, as described above, executable by a combination of algorithms and a widely used computer in accordance with the disclosure as described below.

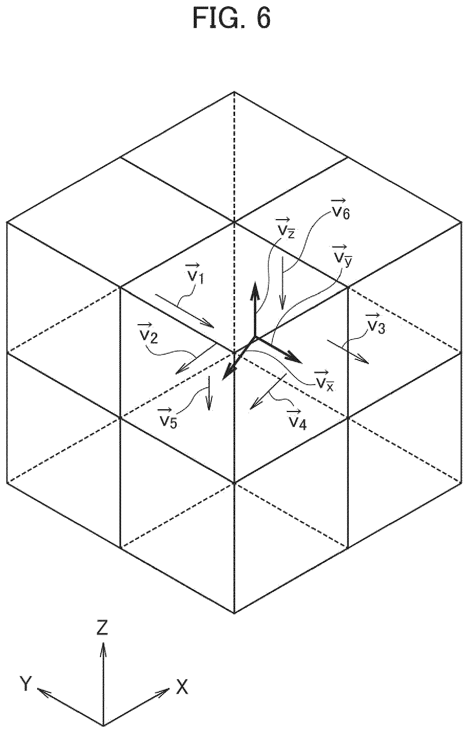

Referring to FIG. 6, the process includes: dividing the totality of the fiber reinforced material in question or any space including a region at issue in the material into proper three-dimensional lattices to set out a plurality of three-dimensional cells. Although FIG. 6 merely shows a small number of vectors in a particular cell, actually a large number of vectors respectively belong to all the cells. From the extracted vector data, data belonging to each cell are sorted out on the basis of coordinates of respective start and end points and are averaged in each cell. $\vec{V} = \frac{\sum \vec{v}}{n}$ (1) $\vec{V} = \frac{\frac{\sum \vec{v}}{n}}{\left|\frac{\sum \vec{v}}{n}\right|}$ (2)

Simple arithmetic averages could be taken in accordance with the equation (1) or these averages could be further normalized in accordance with the equation (2). Or alternatively, each vector could be properly weighted and then weighted averages could be taken. Calculated results will be referred to as "reference vector data" in the following description and the appended claims.

In a case where it is determinable which of the extracted data derives from which of fibers (or fiber bundles) and where the determined fiber is directed in the fabric, on the basis of the information, averages could be taken respectively by vectors related to fibers (or fiber bundles) directed in a particular direction in the fabric before deforming. FIG. 6 shows average vectors after deforming respectively averaged with respect to the X-, Y- and Z-directions. The average vectors are calculated on the basis of the equation (3). $\left.\begin{matrix}\vec{{v}_{\overline{x}}} = average \left({\vec{v}}_{2}, {\vec{v}}_{4}\right) \\ \vec{{v}_{\overline{y}}} = average \left({\vec{v}}_{1}, {\vec{v}}_{3}\right) \\ \vec{{v}_{\overline{z}}} = average \left({\vec{v}}_{5}, {\vec{v}}_{6}\right)\end{matrix}\right\}$ (3)

As will be understood from FIG. 3, a fabric inherently contains many fiber bundles in various positions and in various directions. Further as will be understood from FIG. 4, as the fabric is deformed, these fiber bundles are displaced and also change these directions. Therefore to compare one set of data obtained from one fiber reinforced material with another set of data obtained from another fiber reinforced material is unlikely to give meaningful insights. Because the reference vector data obtained in the present embodiment are representative of directions of fiber bundles in each cell, they are comparable to standard data obtained from similar lattices.

The reference vector data may be, as they are for example, displayed two-dimensionally or three-dimensionally on a display. Unlike raw interior observation results or vector data, the picture on the display enables observation on vectors representative of each cell, and thereby gives considerable insights. For example, it is possible to visually compare it with a stress distribution obtained in numerical calculations or with reference vector data obtained in another opportunity. If the directions of stress in any portion are aligned with the directions of the fiber bundles, it could be expected that this portion is relatively tough. If some misfits exist, the portion is expected to be relatively weak.

For better quantitative comparison, the reference vector data may be further subject to any calculation process, which for example includes: getting differences from the standard data. If reference vector data obtained in another opportunity are adopted as the standard data, the differences represent variations in directions of fiber bundles from one production chance to another and three-dimensional display thereof facilitates understanding as to which regions are likely to create such variations. Of course any standard data are arbitrarily selected and, instead of difference calculation, any available calculation may be used.

Although certain embodiments have been described above, modifications and variations of the embodiments described above will occur to those skilled in the art, in light of the above teachings.

### INDUSTRIAL APPLICABILITY

By calculating the reference vector data obtained by averaging data in each cell, the disclosure enables quantitative comparison among fiber reinforced materials in which fiber bundles are distinctly placed.

## Claims

1. A system for evaluating a distribution of fiber bundles (3, 5, 7) in a fiber reinforced material by three-dimensional vector data of the fiber bundles (3, 5, 7), comprising:
calculator means for dividing the fiber reinforced material into a plurality of three-dimensional cells,
selecting data respectively belonging to the cells, and
averaging the selected data to calculate reference vector data, and
display means for displaying the reference vector data two-dimensionally or three-dimensionally, wherein
the system is **characterized in that**:
it is determinable which of the selected data derives from which of fibers and where the determined fiber is directed in the fabric, and
the reference vector data corresponds to separate average vectors (*̅v̅ₓ̅*̅; *̅v̅ₓ̅*̅; *̅v̅_̅{̅z̅}̅*̅) with respect to the X-, Y- and Z-directions, which are calculated by vectors (*̅v̅*̅₁̅, *̅v̅*̅₂̅,) *̅v̅*̅₃̅*, v*_{4̅}*, v*_{5̅}*, v*_{6̅}) related to the fibers directed in a particular direction in the fabric.

2. The system of claim 1, further comprising:
comparator means for comparing the reference vector data (*̅v̅ₓ̅*̅; *̅v̅_̅{̅y̅}̅*̅; *̅v̅_̅{̅z̅}̅*̅) with other vector data.

3. The system of claim 2, wherein
the comparator means comprises delta display means for calculating differences between the reference vector data (*̅v̅ₓ̅*̅; *̅v̅_̅{̅y̅}̅*̅; *̅v̅_̅{̅z̅}̅*̅) and the other vector data and converting the differences into differences in hue to make the display means display the differences in hue.

## Patentansprüche

1. System zur Auswertung einer Verteilung von Faserbündeln (3, 5, 7) in einem faserverstärkten Material durch dreidimensionale Vektordaten der Faserbündel (3, 5, 7), umfassend:
Rechenmittel zum Unterteilen des faserverstärkten Materials in mehrere dreidimensionale Zellen,
Auswählen von entsprechend zu den Zellen gehörenden Daten und
Mitteln der ausgewählten Daten, um Referenzvektordaten zu berechnen, und
Anzeigemittel zum zweidimensionalen oder dreidimensionalen Anzeigen der Referenzvektordaten, wobei das System **dadurch gekennzeichnet ist, dass**:
es bestimmbar ist, welche der ausgewählten Daten von welcher der Fasern stammen und
wohin die bestimmte Faser in dem Gewebe gerichtet ist, und
die Referenzvektordaten hinsichtlich den Richtungen X, Y und Z separaten Durchschnittsvektoren (*̅v̅ₓ̅*̅, *̅v̅_̅{̅y̅}̅*̅; *̅v̅_̅{̅z̅}̅*̅) entsprechen, die durch Vektoren (*̅v̅*̅₁̅, *̅v̅*̅₂̅, *̅v̅*̅₃̅, *v*_{4̅,} *v*_{5̅}, *v*_{6̅}) in Bezug auf die in einer bestimmten Richtung in dem Gewebe gerichteten Fasern berechnet werden.

2. System nach Anspruch 1, ferner umfassend:
Vergleichsmittel zum Vergleichen der Referenzvektordaten (*̅v̅ₓ̅*̅; *̅v̅_̅{̅y̅}̅*̅; *̅v̅_̅{̅z̅}̅*̅) mit anderen Vektordaten.

3. System nach Anspruch 2, wobei
die Vergleichsmittel Delta-Anzeigemittel zum Berechnen von Differenzen zwischen den Referenzvektordaten $\left(\vec{{v}_{\overline{x}}}; \vec{{v}_{\overline{ν}}}; \vec{{v}_{\overline{z}}}\right)$ und den anderen Vektordaten und zum Umwandeln der Differenzen in Unterschiede im Farbton umfassen, sodass die Anzeigemittel die Unterschiede im Farbton anzeigen.

## Revendications

1. Système d'évaluation de la distribution de faisceaux de fibres (3, 5, 7) dans un matériau renforcé par des fibres par des données vectorielles tridimensionnelles des faisceaux de fibres (3, 5, 7), comprenant :
des moyens de calcul pour diviser le matériau renforcé par des fibres en une pluralité de cellules tridimensionnelles,
la sélection de données appartenant respectivement aux cellules, et
le calcul de la moyenne des données sélectionnées pour calculer des données vectorielles de référence, et
des moyens d'affichage pour afficher les données vectorielles de référence en deux dimensions ou trois dimensions, dans lequel le système est **caractérisé en ce que** :
il est possible de déterminer lesquelles des données sélectionnées proviennent de quelles fibres et
dans quel sens la fibre déterminée est dirigée dans le tissu, et
les données vectorielles de référence correspondent à des vecteurs (v̅ₓ̅; v̅_̅{̅y̅}̅; v̅_̅{̅z̅}̅) moyens séparés par rapport aux directions X, Y et Z, qui sont calculés par des vecteurs (v̅₁̅, v̅₂̅, v̅₃̅, v̅₄̅, v̅₅̅, v̅₆̅) relatifs aux fibres dirigées dans une direction particulière dans le tissu.

2. Système selon la revendication 1, comprenant en outre :
des moyens de comparaison pour comparer les données vectorielles de référence $\left(\vec{{v}_{\hat{x}}}; \vec{{v}_{\hat{y}}}; \vec{{v}_{\hat{z}}}\right)$ à d'autres données vectorielles.

3. Système selon la revendication 2, dans lequel
le moyen de comparaison comprend des moyens d'affichage delta pour calculer des différences entre les données vectorielles de référence $\left(\vec{{v}_{\overline{x}}}; \vec{{v}_{\overline{y}}}; \vec{{v}_{\overline{z}}}\right)$ et les autres données vectorielles et convertir les différences en différences de teinte pour faire en sorte que le moyen d'affichage affiche les différences de teinte.
